# EUROPEAN PATENT APPLICATION

(11) **EP 2 194 435 A2**
(43) Date of publication of application: **09.06.2010**
(21) Application number: 09176819.2
(22) Date of filing: 24.11.2009
(51) Int. Cl.: G05D 1/02

(54) **Garment worn by the operator of a semi-autonomous machine**

(30) Priority: 08.12.2008 US 329930
(71) Applicant: DEERE & COMPANY, Moline, Illinois 61265-8098 (US)
(72) Inventor: Anderson, Noel W., Fargo, ND 58102 (US)
(74) Representative: Holst, Sönke

(57) **Abstract**

A method and apparatus for localizing an operator using a garment is provided. The garment has a number of localization devices capable of being detected by an autonomous vehicle, and a controller capable of sending a control signal to the autonomous vehicle.

## Description

The present disclosure relates generally to systems and methods for machine navigation and more particularly, systems and methods for high integrity coordination of multiple off-road machines. Still more particularly, the present disclosure relates to a method and apparatus for localizing an operator of a machine.

An increasing trend towards developing automated or semi-automated equipment is present in today's work environment. In some situations with the trend, this equipment is completely different from the operator-controlled equipment that is being replaced, and does not allow for any situations in which an operator can be present near the machine or take over operation of the machine. Such unmanned equipment can be unreliable due to the complexity of systems involved, the current status of computerized control, and uncertainty in various operating environments. As a result, semi-automated equipment is more commonly used. This type of equipment is similar to previous operator-controlled equipment, but incorporates one or more operations that are automated rather than operator-controlled. This semi-automated equipment allows for human supervision and allows the operator to take control when necessary.

The illustrative embodiments provide a method and apparatus for localizing an operator using a garment, a number of localization devices capable of being detected by an autonomous machine, and a controller capable of sending a control signal to the autonomous machine.

The features, functions, and advantages can be achieved independently in various embodiments of the present invention or may be combined in yet other embodiments in which further details can be seen with reference to the following description and drawings, wherein:
Figure 1 is a block diagram of a worker and a vehicle in an operating environment in which an illustrative embodiment may be implemented;
Figure 2 is a block diagram of a machine interacting with an operator in accordance with an illustrative embodiment;
Figure 3 is a block diagram of a garment in accordance with an illustrative embodiment;
Figure 4 is a block diagram of a data processing system in accordance with an illustrative embodiment;
Figure 5 is a block diagram of functional software components that may be implemented in a machine controller in accordance with an illustrative embodiment;
Figure 6 is a block diagram of components used to control a vehicle in accordance with an illustrative embodiment;
Figure 7 is a block diagram of a knowledge base in accordance with an illustrative embodiment;
Figure 8 is a block diagram of a fixed knowledge base in accordance with an illustrative embodiment;
Figure 9 is a block diagram of a learned knowledge base in accordance with an illustrative embodiment;
Figure 10 is a block diagram of a format in a knowledge base used to select sensors for use in detecting and localizing a garment and/or worker in accordance with an illustrative embodiment;
Figure 11 is a flowchart illustrating a process for engaging a vehicle in accordance with an illustrative embodiment;
Figure 12 is a flowchart illustrating a process for authenticating a worker in accordance with an illustrative embodiment;
Figure 13 is a flowchart illustrating a process for localization of a worker by a vehicle in accordance with an illustrative embodiment;
Figure 14 is a flowchart illustrating a process for controlling a vehicle with a garment in accordance with an illustrative embodiment;
Figure 15 is a flowchart illustrating a process for receiving commands from a garment to control a vehicle in accordance with an illustrative embodiment;
Figure 16 is a flowchart illustrating a process for monitoring the condition of a worker in accordance with an illustrative embodiment;
Figure 17 is a flowchart illustrating a process for monitoring the condition of the operating environment in accordance with an illustrative embodiment; and
Figure 18 is a flowchart illustrating a process for side-following in accordance with an illustrative embodiment.

Robotic or autonomous machines, sometimes referred to as mobile robotic platforms, generally have a robotic control system that controls the operational systems of the machine. In a machine that is limited to a transportation function, such as a vehicle for example, the operational systems may include steering, braking, transmission, and throttle systems. Such autonomous machines generally have a centralized robotic control system for control of the operational systems of the machine. Some military vehicles have been adapted for autonomous operation. In the United States, some tanks, personnel carriers, Stryker vehicles, and other vehicles have been adapted for autonomous capability. Generally, these are to be used in a manned mode as well.

Robotic control system sensor inputs may include data associated with the machine's destination, preprogrammed path information, and detected obstacle information. Based on such data associated with the information above, the machine's movements are controlled. Obstacle detection systems within a machine commonly use scanning lasers to scan a beam over a field of view, or cameras to capture images over a field of view. The scanning laser may cycle through an entire range of beam orientations, or provide random access to any particular orientation of the scanning beam. The camera or cameras may capture images over the broad field of view, or of a particular spectrum within the field of view. For obstacle detection applications of a machine, the response time for collecting image data should be rapid over a wide field of view to facilitate early recognition and avoidance of obstacles.

Location sensing devices include odometers, global positioning systems, and vision-based triangulation systems. Many location sensing devices are subject to errors in providing an accurate location estimate over time and in different geographic positions. Odometers are subject to material errors due to surface terrain. Satellite-based guidance systems, such as global positioning system-based guidance systems, which are commonly used today as a navigation aid in cars, airplanes, ships, computer-controlled harvesters, mine trucks, and other vehicles, may experience difficulty guiding when heavy foliage or other permanent obstructions, such as mountains, buildings, trees, and terrain, prevent or inhibit global positioning system signals from being accurately received by the system. Vision-based triangulation systems may experience error over certain angular ranges and distance ranges because of the relative position of cameras and landmarks.

In order to provide a system and method where multiple combination manned/autonomous machines accurately navigate and manage a work-site alongside human operators, specific mechanical accommodations for processing means and location sensing devices are required. Therefore, it would be advantageous to have a method and apparatus to provide additional features for navigation and coordination of multiple machines.

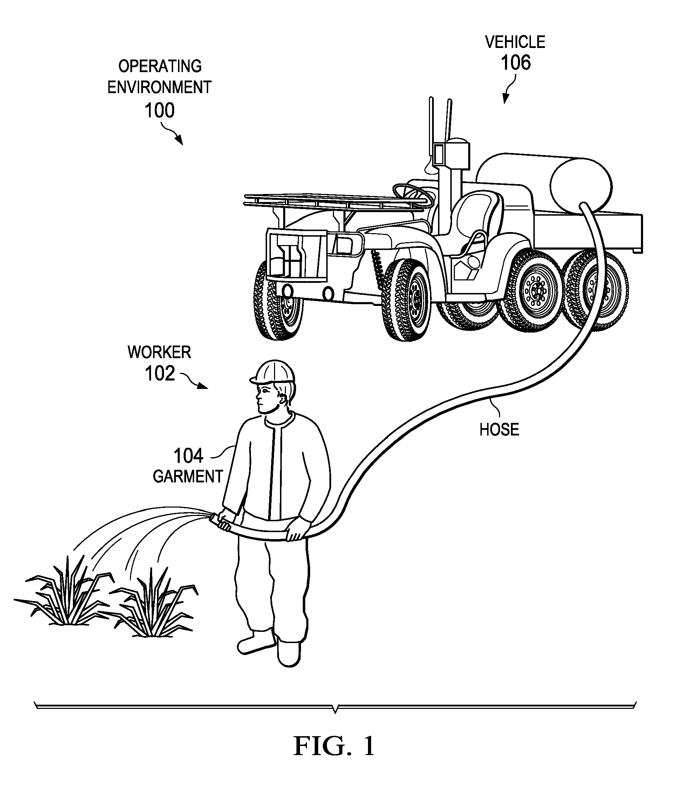

The illustrative embodiments recognize a need for a system and method where multiple combination manned/autonomous machines can accurately navigate and manage a work-site alongside human operators. Therefore, the illustrative embodiments provide a computer implemented method, apparatus, and computer program product for coordinating machines and localizing workers using a garment worn by a human operator. With reference to the figures and in particular with reference to Figure 1, different illustrative embodiments may be used in a variety of different machines, such as vehicles, machines in a production line, and other machine operating environments. For example, a machine in a production line may be a robot that welds parts on an assembly line. For example, the different illustrative embodiments may be used in a variety of vehicles, such as automobiles, trucks, harvesters, combines, agricultural equipment, tractors, mowers, armored vehicles, and utility vehicles. Embodiments of the present invention may also be used in a single computing system or a distributed computing system.

The illustration of a vehicle or vehicles provided in the following figures is not meant to imply physical or architectural limitations to the manner in which different illustrative embodiments may be implemented. For example, in other embodiments, other machines may be used in addition to or in place of the vehicles depicted in these figures. For example, in other embodiments, vehicle 106 in Figure 1 may be a machine in a production assembly line.

Figure 1 depicts a block diagram of a worker and a vehicle in an operating environment in accordance with an illustrative embodiment. A worker is one illustrative example of an operator that may work in coordination with a vehicle in an operating environment. A number of items, as used herein, refer to one or more items. For example, a number of workers is one or more workers. The illustrative embodiments may be implemented using a number of vehicles and a number of operators. Figure 1 depicts an illustrative environment including operating environment 100 in one embodiment. In this example, operating environment 100 may be any type of work-site with vegetation, such as, for example, bushes, flower beds, trees, grass, crops, or other foliage.

In this example, vehicle 106 may be any type of autonomous or semi-autonomous utility vehicle used for spraying, fertilizing, watering, or cleaning vegetation. Vehicle 106 may perform operations independently of the operator, simultaneously with the operator, or in a coordinated manner with the operator or with other autonomous or semi-autonomous vehicles. In this illustrative embodiment, vehicle 106 may have a chemical sprayer mounted and follow an operator, such as worker 102, wearing a garment, such as garment 104, as the operator applies chemicals to crops or other foliage In this example, worker 102 may be any type of operator. In the illustrative examples, an operator is defined as the wearer of the garment. An operator may include, without limitation, a human, animal, robot, instance of an autonomous vehicle, or any other suitable operator. In this example, garment 104 may be any type of garment worn by an operator, such as worker 102.

Vehicle 106 and garment 104 operate in a coordinated manner using high integrity systems. As used herein, "high integrity" when used to describe a component means that the component performs well across different operating environments. In other words, as the external environment changes to reduce the capability of components in a system or a component internally fails in the system, a level of redundancy is present in the number and the capabilities of remaining components to provide fail-safe or preferably fail-operational perception of the environment without human monitoring or intervention.

Sensors, wireless links, and actuators are examples of components that may have a reduced capability in different operating environments. For example, a wireless communications link operating in one frequency range may not function well if interference occurs in the frequency range, while another communications link using a different frequency range may be unaffected. In another example, a high integrity coordination system has hardware redundancy that allows the system to continue to operate. The level of operation may be the same. The level of operation may be at a reduced level after some number of failures in the system, such that a failure of the system is graceful.

A graceful failure means that a failure in a system component will not cause the system to fail entirely or immediately stop working. The system may lose some level of functionality or performance after a failure of a hardware and/or software component, an environmental change, or from some other failure or event. The remaining level and duration of functionality may only be adequate to bring the vehicle to a safe shutdown. On the other end of the spectrum, full functionality may be maintainable until another component failure.

In an illustrative example, vehicle 106 may be a follower vehicle and garment 104 may be the leader. Vehicle 106 may operate in operating environment 100 following garment 104 using a number of different modes of operation to aid an operator in spraying, fertilizing, watering, or cleaning vegetation. A number of items as used herein refer to one or more items. For example, a number of different modes is one or more different modes. In another illustrative example, vehicle 106 may coordinate its movements in order to execute a shared task at the same time as worker 102, or another vehicle operating in the worksite, for example, moving alongside worker 102 as worker 102 sprays fertilizer onto vegetation using a hose connected to vehicle 106. The modes include, for example, a side following mode, a teach and playback mode, a teleoperation mode, a path mapping mode, a straight mode, and other suitable modes of operation. An operator may be, for example, a person being followed as the leader when the vehicle is operating in a side-following mode, a person driving the vehicle, and/or a person controlling the vehicle movements in teleoperation mode.

In one example, in the side following mode, an operator wearing garment 104 is the leader and vehicle 106 is the follower. In one illustrative embodiment, vehicle 106 may be one of multiple vehicles that are followers, following worker 102, wearing garment 104, in a coordinated manner to perform a task in operating environment 100.

The side following mode may include preprogrammed maneuvers in which an operator may change the movement of vehicle 106 from an otherwise straight travel path for vehicle 106. For example, if an obstacle is detected in operating environment 100, the operator may initiate a go around obstacle maneuver that causes vehicle 106 to steer out and around an obstacle in a preset path.

With this mode, automatic obstacle identification and avoidance features may still be used. The different actions taken by vehicle 106 may occur with the aid of machine control component in accordance with an illustrative embodiment. The machine control component used by vehicle 106 may be located within vehicle 106 and/or located remotely from vehicle 106 in a garment, such as garment 104. In some embodiments, the machine control component may be distributed between a vehicle and a garment or between a number of vehicles and a garment.

In another example, an operator may drive vehicle 106 along a path in operating environment 100 without stops, generating a mapped path. After driving the path, the operator may move vehicle 106 back to the beginning of the mapped path, and assign a task to vehicle 106 using the mapped path generated while driving vehicle 106 along the path. In the second pass of the path, the operator may cause vehicle 106 to drive the mapped path from start point to end point without stopping, or may cause vehicle 106 to drive the mapped path with stops along the mapped path.

In this manner, vehicle 106 drives from start to finish along the mapped path. Vehicle 106 still may include some level of obstacle detection to keep vehicle 106 from running over or hitting an obstacle, such as worker 102 or another vehicle in operating environment 100. These actions also may occur with the aid of a machine control component in accordance with an illustrative embodiment.

In a teleoperation mode, for example, an operator may operate or wirelessly control vehicle 106 using controls located on garment 104 in a fashion similar to other remote controlled vehicles. With this type of mode of operation, the operator may control vehicle 106 through a wireless controller.

In a path mapping mode, the different paths may be mapped by an operator prior to reaching operating environment 100. With a fertilizing example, paths may be identical for each pass of a section of vegetation and the operator may rely on the fact that vehicle 106 will move along the same path each time. Intervention or deviation from the mapped path may occur only when an obstacle is present. Also, in an illustrative embodiment, with the path mapping mode, way points may be set to allow vehicle 106 to stop at various points.

In a straight mode, vehicle 106 may be placed in the middle or offset from some distance from an edge of a path. Vehicle 106 may move down the path along a straight line. In this type of mode of operation, the path of vehicle 106 is always straight unless an obstacle is encountered. In this type of mode of operation, the operator may start and stop vehicle 106 as needed. This type of mode may minimize the intervention needed by a driver. Some or all of the different operations in these examples may be performed with the aid of a machine control component in accordance with an illustrative embodiment.

In different illustrative embodiments, the different types of mode of operation may be used in combination to achieve the desired goals. In these examples, at least one of these modes of operation may be used to minimize driving while maximizing safety and efficiency in a fertilizing process. In these examples, the vehicle depicted may utilize each of the different types of mode of operation to achieve desired goals. As used herein, the phrase "at least one of" when used with a list of items means that different combinations of one or more of the items may be used and only one of each item in the list may be needed. For example, "at least one of item A, item B, and item C" may include, for example, without limitation, item A or item A and item B. This example also may include item A, item B, and item C or item B and item C. As another example, at least one of item A, item B, and item C may include item A, two of item B, and 4 of item C.

In different illustrative embodiments, dynamic conditions impact the movement of a vehicle. A dynamic condition is a change in the environment around a vehicle. For example, a dynamic condition may include, without limitation, movement of another vehicle in the environment to a new location, detection of an obstacle, detection of a new object or objects in the environment, receiving user input to change the movement of the vehicle, receiving instructions from a control system, such as garment 104, system or component failure in a vehicle, and the like. In response to a dynamic condition, the movement of a vehicle may be altered in various ways, including, without limitation, stopping the vehicle, accelerating propulsion of the vehicle, decelerating propulsion of the vehicle, and altering the direction of the vehicle, for example.

Further, autonomous routes may include several straight blocks. In other examples, a path may go around blocks in a square or rectangular pattern. Of course, other types of patterns also may be used depending upon the particular implementation. Routes and patterns may be performed with the aid of a knowledge base in accordance with an illustrative embodiment. In these examples, an operator may drive vehicle 106 onto a field or to a beginning position of a path. The operator also may monitor vehicle 106 for safe operation and ultimately provide overriding control for the behavior of vehicle 106.

In these examples, a path may be a preset path, a path that is continuously planned with changes made by vehicle 106 to follow an operator in a side following mode, a path that is directed by the operator using a remote control in a teleoperation mode, or some other path. The path may be any length depending on the implementation. Paths may be stored and accessed with the aid of a knowledge base in accordance with an illustrative embodiment.

In these examples, heterogeneous sets of redundant sensors are located on the vehicle and on the garment in a worksite to provide high integrity perception with fault tolerance. Redundant sensors in these examples are sensors that may be used to compensate for the loss and/or inability of other sensors to obtain information needed to control a vehicle or detect a worker. A redundant use of the sensor sets are governed by the intended use of each of the sensors and their degradation in certain dynamic conditions. The sensor sets robustly provide data for localization and/or safeguarding in light of a component failure or a temporary environmental condition. For example, dynamic conditions may be terrestrial and weather conditions that affect sensors and their ability to contribute to localization and safeguarding. Such conditions may include, without limitation, sun, clouds, artificial illumination, full moon light, new moon darkness, degree of sun brightness based on sun position due to season, shadows, fog, smoke, sand, dust, rain, snow, and the like.

In these examples, heterogeneous sets of redundant vehicle control components are located on the vehicle and the garment in a worksite to provide high integrity machine control with fault tolerance. Redundant vehicle control components in these examples are vehicle control components that may be used to compensate for the loss and/or inability of other vehicle control components to accurately and efficiently control a vehicle. For example, redundant actuators controlling a braking system may provide for fault tolerance if one actuator malfunctions, enabling another actuator to maintain control of the braking system for the vehicle and providing high integrity to the vehicle control system.

In these examples, heterogeneous sets of communication links and channels are located on the vehicle and the garment in a worksite to provide high integrity communication with fault tolerance. Redundant communication links and channels in these examples are communication links and channels that may be used to compensate for the loss and/or inability of other communication links and channels to transmit or receive data to or from a vehicle and a garment. Multiple communications links and channels may provide redundancy for fail-safe communications. For example, redundant communication links and channels may include AM radio frequency channels, FM radio frequency channels, cellular frequencies, global positioning system receivers, Bluetooth receivers, Wi-Fi channels, and Wi-Max channels.

In these examples, redundant processors are located on the vehicle in a worksite to provide high integrity machine coordination with fault tolerance. The high integrity machine coordination system may share the physical processing means with the high integrity machine control system or have its own dedicated processors.

Thus, the different illustrative embodiments provide a number of different modes to operate a vehicle, such as vehicle 106, using a garment, such as garment 104. Although Figure 1 illustrates a vehicle for spraying, fertilizing, watering, or cleaning vegetation, this illustration is not meant to limit the manner in which different modes may be applied. For example, the different illustrative embodiments may be applied to other types of vehicles and other types of uses. In an illustrative example, different types of vehicles may include controllable vehicles, autonomous vehicles, semi-autonomous vehicles, or any combination thereof.

Vehicles may include vehicles with legs, vehicles with wheels, vehicles with tracks, vehicles with rails, and vehicles with rollers. As a specific example, the different illustrative embodiments may be applied to a military vehicle in which a soldier uses a side following mode to provide a shield across a clearing. In other embodiments, the vehicle may be an agricultural vehicle used for harvesting, threshing, or cleaning crops. In another example, illustrative embodiments may be applied to golf and turf care vehicles. In still another example, the embodiments may be applied to forestry vehicles having functions, such as felling, bucking, forwarding, or other suitable forestry applications. These types of modes also may provide obstacle avoidance and remote control capabilities. As yet another example, the different illustrative embodiments may be applied to delivery vehicles, such as those for the post office or other commercial delivery vehicles.

In addition, the different illustrative embodiments may be implemented in any number of vehicles. For example, the different illustrative embodiments may be implemented in as few as one vehicle, or in two or more vehicles, or any number of multiple vehicles. Further, the different illustrative embodiments may be implemented in a heterogeneous group of vehicles or in a homogeneous group of vehicles. As one example, the illustrative embodiments may be implemented in a group of vehicles including a personnel carrier, a tank, and a utility vehicle. In another example, the illustrative embodiments may be implemented in a group of six utility vehicles.

The different illustrative embodiments may be implemented using any number of operators. For example, the different illustrative embodiments may be implemented using one operator, two operators, or any other number of operators. The different illustrative embodiments may be implemented using any combination of any number of vehicles and operators. As one example, the illustrative embodiments may be implemented using one vehicle and one operator. In another example, the illustrative embodiments may be implemented using one vehicle and multiple operators. In yet another example, the illustrative embodiments may be implemented using multiple vehicles and multiple operators. In yet another example, the illustrative embodiments may be implemented using multiple vehicles and one operator.

The description of the different illustrative embodiments has been presented for purposes of illustration and description, and is not intended to be exhaustive or limited to the embodiments in the form disclosed. Many modifications and variations will be apparent to those of ordinary skill in the art. Further, different embodiments may provide different advantages as compared to other embodiments. The embodiment or embodiments selected are chosen and described in order to best explain the principles of the invention, the practical application, and to enable others of ordinary skill in the art to understand the invention for various embodiments with various modifications as are suited to the particular use contemplated.

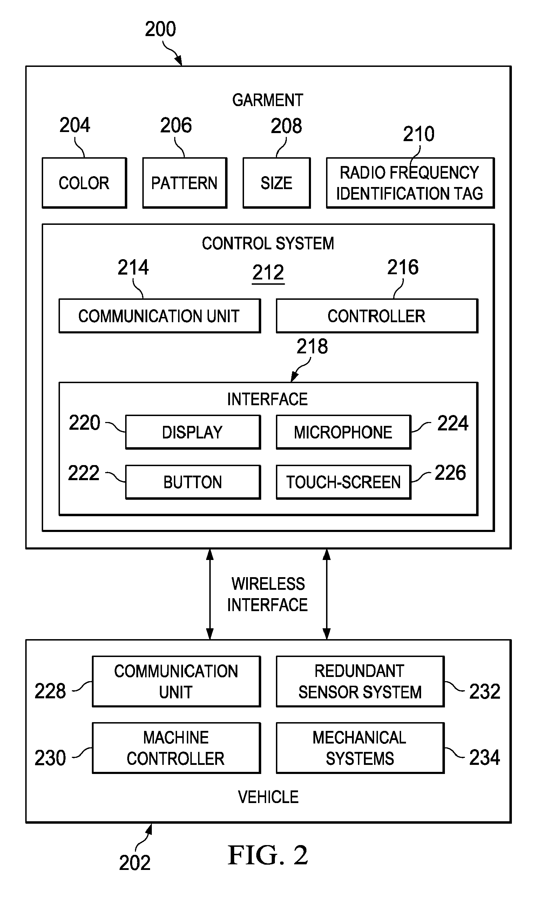

With reference now to Figure 2, a block diagram of a machine interacting with an operator is depicted in accordance with an illustrative embodiment. Garment 200 is an example of garment 104 in Figure 1. Garment 200 may be any type of garment including, without limitation, a vest, a jacket, a helmet, a shirt, a jumpsuit, a glove, and the like. Vehicle 202 is an example of vehicle 106 in Figure 1.

Garment 200 includes color 204, pattern 206, size 208, radio frequency identification tag 210, and control system 212. Color 204 may be, without limitation, the color of the garment material or a color block located on the garment.

Pattern 206 may be, without limitation, a visible logo, a visible symbol, a barcode, or patterned garment material. Size 208 may be, without limitation, the size of the garment, or the size of a visible area of the garment. Color 204, pattern 206, and size 208 may be used to identify the wearer of garment 200 and well as localize the wearer.

Radio frequency identification tag 210 stores and processes information, as well as transmits and receives a signal through a built-in antennae. Radio frequency identification tag 210 is detected by a radio frequency identification reader located in a sensor system, such as redundant sensor system 232 on vehicle 202. Radio frequency identification tag 210 may operate on a number of different frequencies to provide high integrity to the detection of garment 200. Garment 200 may have a number of radio frequency identification tags. As used herein, a number may be one or more frequencies, or one or more radio frequency identification tags.

Control system 212 includes communication unit 214, controller 216, and interface 218. Communication unit 214, in these examples, provides for communications with other data processing systems or devices, such as communications unit 228 located on vehicle 202. In these examples, communication units 214 and 228 include multiple communications links and channels in order to provide redundancy for fail-safe communications. For example, communication units 214 and 228 may communicate using AM radio frequency transceivers, FM radio frequency transceivers, cellular unit, global positioning system receivers, Bluetooth receivers, Wi-Fi transceivers, and Wi-Max transceivers. Communication units 214 and 228 may provide communications through the use of either or both physical and wireless communications links.

Controller 216 may be implemented using a processor or similar device. Controller 216 receives user input from interface 218, generates commands, and transmits the commands to machine controller 230 in vehicle 202. In an illustrative embodiment, controller 216 may transmit commands to machine controller 230 through communication unit 214 by emitting a radio frequency that can be detected by communication unit 228 on vehicle 202. Controller 216 can also receive information from machine controller 230 in vehicle 202. In an illustrative embodiment, controller 216 may also be integrated with touchscreen 226.

Interface 218 includes display 220, button 222, microphone 224, and touchscreen 226. Display 220 may be a display screen affixed to or integrated in the garment, visible to an operator. Display 220 provides a user interface for viewing information sent to garment 200 by vehicle 202. Button 222 may be any type of button used to transmit a signal or command to vehicle 202. For example, in an illustrative embodiment, button 222 may be an emergency stop button. In an illustrative embodiment, if multiple vehicles are in the operating environment, an emergency stop button may also include a selection option to select vehicle 202 for the emergency stop command. Microphone 224 may be any type of sensor that converts sound into an electrical signal. In an illustrative embodiment, microphone 224 may detect the voice of an operator, such as worker 102 in Figure 1, and convert the sound of the operator's voice into an electrical signal transmitted to a receiver on vehicle 202. Microphone 224 may allow an operator, such as worker 102 in Figure 1, to control a vehicle, such as vehicle 106 in Figure 1, using voice commands.

Touchscreen 226 is an area that can detect the presence and location of a touch within the area. In an illustrative embodiment, touchscreen 226 may detect a touch or contact to the area by a finger or a hand. In another illustrative embodiment, touchscreen 226 may detect a touch or contact to the area by a stylus, or other similar object. Touchscreen 226 may contain control options that allow an operator, such as worker 102 in Figure 1, to control a vehicle, such as vehicle 106 in Figure 1, with the touch of a button or selection of an area on touchscreen 226. Examples of control options may include, without limitation, propulsion of the vehicle, accelerating the propulsion of the vehicle, decelerating propulsion of the vehicle, steering the vehicle, braking the vehicle, and emergency stop of the vehicle. In an illustrative embodiment, touchscreen 226 may be integrated with controller 216. In another illustrative embodiment, controller 216 may be manifested as touchscreen 226.

Vehicle 202 includes communication unit 228, machine controller 230, redundant sensor system 232, and mechanical system 234.

Communication unit 228 in these examples provides for communications with other data processing systems or devices, such as communications unit 214 located on garment 200. In these examples, communication unit 228 includes multiple communications links and channels in order to provide redundancy for fail-safe communications. For example, communication unit 228 may include AM radio frequency transceivers, FM radio frequency transceivers, cellular unit, global positioning system receivers, Bluetooth receivers, Wi-Fi transceivers, and Wi-Max transceivers. Communication unit 228 may provide communications through the use of either or both physical and wireless communications links.

Machine controller 230 may be, for example, a data processing system or some other device that may execute processes to control movement of a vehicle. Machine controller 230 may be, for example, a computer, an application integrated specific circuit, and/or some other suitable device. Different types of devices and systems may be used to provide redundancy and fault tolerance. Machine controller 230 may execute processes using high integrity control software to control mechanical system 234 in order to control movement of vehicle 202. Machine controller 230 may send various commands to mechanical system 234 to operate vehicle 202 in different modes of operation. These commands may take various forms depending on the implementation. For example, the commands may be analog electrical signals in which a voltage and/or current change is used to control these systems. In other implementations, the commands may take the form of data sent to the systems to initiate the desired actions.

Redundant sensor system 232 is a high integrity sensor system and may be a set of sensors used to collect information about the environment around a vehicle and the people in the environment around the vehicle. Redundant sensor system 232 may detect color 204, pattern 206, size 208, radio frequency identification tag 210 on garment 200, and use the detected information to identify and localize the wearer of garment 200. In these examples, the information is sent to machine controller 230 to provide data in identifying how the vehicle should move in different modes of operation in order to safely operate in the environment with the wearer of garment 200. In these examples, a set refers to one or more items. A set of sensors is one or more sensors in these examples. A set of sensors may be a heterogeneous and/or homogeneous set of sensors.

In an illustrative embodiment, redundant sensor system 232 detects an obstacle in the operating environment of vehicle 202, and sends information about the obstacle to display 220 on garment 200. The operator wearing garment 200 views the information and uses touchscreen 226 to send an obstacle avoidance command back to vehicle 202. In another illustrative embodiment, redundant sensor system 232 detects an obstacle in the operating environment of vehicle 202 and automatically executes obstacle avoidance maneuvers. In yet another illustrative embodiment, redundant sensor system 232 detects an obstacle in the operating environment of vehicle 202, sends information about the obstacle detection to display 220 on garment 200, and automatically executes obstacle avoidance maneuvers without receiving an obstacle avoidance command from the operator.

Mechanical system 234 may include various vehicle control components such as, without limitation, steering systems, propulsion systems, and braking systems. Mechanical system 234 receives commands from machine controller 230.

In an illustrative example, an operator wearing garment 200 uses touchscreen 226 to send a braking command to machine controller 230 in vehicle 202. Machine controller 230 receives the command, and interacts with mechanical system 234 to apply the brakes of vehicle 202.

The illustration of garment 200 is not meant to imply physical or architectural limitations to the manner in which different illustrative embodiments may be implemented. For example, in other embodiments, other components may be used in addition to or in place of the ones illustrated for garment 200. For example, in other embodiments, garment 200 may not have display 220. In still other illustrative embodiments, garment 200 may include a network to interconnect different devices. Also, in other embodiments, garment 200 may include a personal digital assistant, a mobile phone, or some other suitable device. In yet other embodiments, control system 212 may take the form of an emergency stop button and a transmitter.

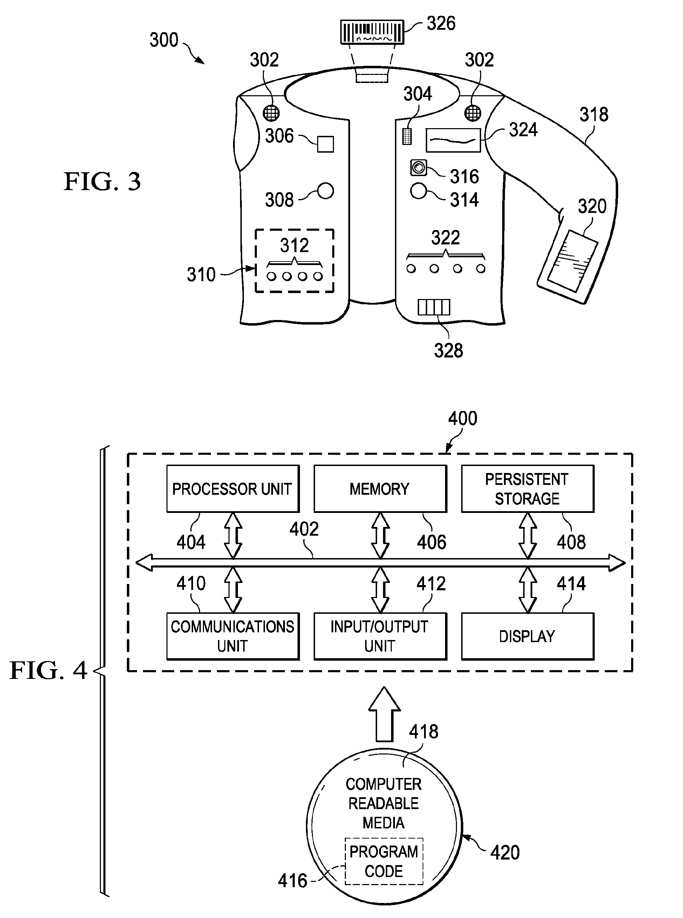

Figure 3 is a block diagram of a garment in accordance with an illustrative embodiment. Garment 300 is an example of garment 104 in Figure 1. Garment 300 is also an example of a manner in which garment 200 in Figure 2 may be implemented.

Garment 300 includes speakers 302, microphone 304, wireless communications module 306, radio frequency identification tag 308, touch sensitive area 310, touch sensors 312, global positioning system sensor 314, camera 316, sleeve 318, display 320, redundant sensors 322, visual logo 324, barcode 326, and battery pack 328. Speakers 302 may be any type of electromechanical transducer that converts an electrical signal to sound. There may be one or more of speakers 302 on garment 300. In an illustrative embodiment, speakers 302 may receive an electrical signal from a vehicle, such as vehicle 202 in Figure 2, carrying information about the vehicle, the worksite, the task, or the worker.

Microphone 304 may be any type of sensor that converts sound into an electrical signal. In an illustrative embodiment, microphone 304 may detect the voice of an operator, such as worker 102 in Figure 1, and convert the sound of the operator's voice into an electrical signal transmitted to a receiver on a vehicle, such as vehicle 106 in Figure 1.

Wireless communications module 306 is an example of communications unit 214 in control system 212 of Figure 2. Wireless communications module 306 allows for wireless communication between garment 300 and a vehicle in the same worksite. Wireless communications module 306 may be a set of redundant homogeneous and/or heterogeneous communication channels. A set of communication channels may include multiple communications links and channels in order to provide redundancy for fail-safe communications. For example, wireless communications module 306 may include AM radio frequency channels, FM radio frequency channels, cellular frequencies, global positioning system receivers, Bluetooth receivers, Wi-Fi channels, and Wi-Max channels.

Radio frequency identification tag 308 is one example of radio frequency identification tag 210 in Figure 2. Radio frequency identification tag 308 stores and processes information, as well as transmits and receives a signal through a built-in antennae. Radio frequency identification tag 308 is detected by a radio frequency identification reader located in a sensor system, such as redundant sensor system 232 on vehicle 202 in Figure 2, which enables vehicle 202 to detect and localize the presence and orientation of the wearer of garment 300.

Touch sensitive area 310 is one example of touchscreen 226 in Figure 2. Touch sensitive area 310 includes touch sensors 312, which can detect the presence and location of a touch. In an illustrative embodiment, touch sensors 312 of touch sensitive area 310 may detect a touch or contact to the area by a finger or a hand. In another illustrative embodiment, touch sensors 312 may detect a touch or contact to the area by a stylus, or other similar object. Touch sensors 312 may each be directed to a different control option that allows an operator, such as worker 102 in Figure 1, to control a vehicle, such as vehicle 106 in Figure 1, with the touch of one of the sensors of touch sensors 312. In an illustrative embodiment, touch sensors 312 may include, without limitation, control for propulsion of the vehicle, accelerating the propulsion of the vehicle, decelerating propulsion of the vehicle, steering the vehicle, braking the vehicle, and emergency stop of the vehicle.

Global positioning system sensor 314 may identify the location of garment 300 with respect to other objects in the environment, including one or more vehicles. Global positioning system sensor 314 may also provide a signal to a vehicle in the worksite, such as vehicle 106 in Figure 1, to enable the vehicle to detect and localize the worker wearing garment 300. Global positioning system sensor 314 may be any type of radio frequency triangulation scheme based on signal strength and/or time of flight. Examples include, without limitation, the Global Positioning System, Glonass, Galileo, and cell phone tower relative signal strength. Position is typically reported as latitude and longitude with an error that depends on factors, such as ionispheric conditions, satellite constellation, and signal attenuation from vegetation.

Camera 316 may be any type of camera including, without limitation, an infrared camera or visible light camera. Camera 316 6 may be one camera, or two or more cameras. Camera 316 may be a set of cameras including two or more heterogeneous and/or homogeneous types of camera. An infrared camera detects heat indicative of a living thing versus an inanimate object. An infrared camera may also form an image using infrared radiation. A visible light camera may be a standard still-image camera, which may be used alone for color information or with a second camera to generate stereoscopic or three-dimensional images. When a visible light camera is used along with a second camera to generate stereoscopic images, the two or more cameras may be set with different exposure settings to provide improved performance over a range of lighting conditions. A visible light camera may also be a video camera that captures and records moving images.

Sleeve 318 is one illustrative embodiment of an optional portion of garment 300, where garment 300 is a vest. Garment 300 may be any type of garment including, without limitation, a vest, a jacket, a helmet, a shirt, a jumpsuit, a glove, and the like. Garment 300 may have optional portions or features, such as sleeve 318, for example. In another illustrative embodiment, garment 300 may be a shirt with an optional feature of long or short sleeves. In yet another illustrative embodiment, garment 300 may be a glove with an optional feature of finger enclosures. The illustrative embodiments provided are not meant to limit the physical architecture of garment 300 in any way.

Display 320 may be a display screen affixed to or integrated in garment 300, visible to an operator. Display 320 provides a user interface for viewing information sent to garment 300 by a vehicle, such as vehicle 202 in Figure 2.

Redundant sensors 322 may be any type of sensors used for monitoring the environment around garment 300 and/or the well-being of the wearer of garment 300. Examples of redundant sensors 322 may include, without limitation, a heart-rate monitor, a blood pressure sensor, a CO₂ monitor, a body temperature sensor, an environmental temperature sensor, a hazardous chemical sensor, a toxic gas sensor, and the like.

Visual logo 324 may be any type of logo visible to a camera on a vehicle, such as vehicle 106 in Figure 1. Visual logo 324 may be a company logo, a company name, a symbol, a word, a shape, or any other distinguishing mark visible on garment 300. Visual logo 324 is detected by a visible light camera on a vehicle, and used to identify the wearer of garment 300 as well as localize the wearer of garment 300 and determine his or her orientation.

Barcode 326 may be any type of an optical machine-readable representation of data. Barcode 326 may be readable by a barcode scanner located on a vehicle or hand-held by an operator. Battery pack 328 may be any type of array of electrochemical cells for electricity storage, or one electrochemical cell for electricity storage. Battery pack 328 may be disposable or rechargeable.

In an illustrative embodiment, garment 300 is used by an operator to control the movement of a vehicle in performing a task in a work-site. In one illustrative embodiment, the work-site is an area of flower beds and the task is applying a chemical spray to the flower beds. The operator may wear garment 300. Radio frequency identification tag 306 allows the vehicle with the chemical spray tank, such as vehicle 106 in Figure 1, to detect and perform localization of garment 300 in order to work alongside the operator wearing garment 300. In one illustrative embodiment, the operator may speak a voice command to control movement of the vehicle, which is picked up by microphone 304 and converted into an electrical signal transmitted to the vehicle. In another illustrative embodiment, the operator may use touch sensitive area 310 to control the movement of the vehicle, selecting a command option provided by one of touch sensors 312 in order to transmit a command to the machine controller of the vehicle, such as machine controller 230 in Figure 2. The vehicle will move according to the command in order to execute the task while maintaining awareness of garment 300 using a sensor system, such as redundant sensor system 232 in Figure 2. This allows for high integrity coordination between a vehicle and the operator wearing garment 300 to ensure safety and provide fail-safe operational work conditions for a human operator.

With reference now to Figure 4, a block diagram of a data processing system is depicted in accordance with an illustrative embodiment. Data processing system 400 is an example of one manner in which the interaction between garment 104 and vehicle 106 in Figure 1 may be implemented. In this illustrative example, data processing system 400 includes communications fabric 402, which provides communications between processor unit 404, memory 406, persistent storage 408, communications unit 410, input/output (I/O) unit 412, and display 414.

Processor unit 404 serves to execute instructions for software that may be loaded into memory 406. Processor unit 404 may be a set of one or more processors or may be a multi-processor core, depending on the particular implementation. Further, processor unit 404 may be implemented using one or more heterogeneous processor systems in which a main processor is present with secondary processors on a single chip. As another illustrative example, processor unit 404 may be a symmetric multi-processor system containing multiple processors of the same type.

Memory 406 and persistent storage 408 are examples of storage devices. A storage device is any piece of hardware that is capable of storing information either on a temporary basis and/or a permanent basis. Memory 406, in these examples, may be, for example, a random access memory or any other suitable volatile or non-volatile storage device. Persistent storage 408 may take various forms depending on the particular implementation. For example, persistent storage 408 may contain one or more components or devices. For example, persistent storage 408 may be a hard drive, a flash memory, a rewritable optical disk, a rewritable magnetic tape, or some combination of the above. The media used by persistent storage 408 also may be removable. For example, a removable hard drive may be used for persistent storage 408.

Communications unit 410, in these examples, provides for communications with other data processing systems or devices. In these examples, communications unit 410 is a network interface card. Communications unit 410 may provide communications through the use of either or both physical and wireless communications links.

Input/output unit 412 allows for input and output of data with other devices that may be connected to data processing system 400. For example, input/output unit 412 may provide a connection for user input through a keyboard and mouse. Further, input/output unit 412 may send output to a printer. Display 414 provides a mechanism to display information to a user.

Instructions for the operating system and applications or programs are located on persistent storage 408. These instructions may be loaded into memory 406 for execution by processor unit 404. The processes of the different embodiments may be performed by processor unit 404 using computer implemented instructions, which may be located in a memory, such as memory 406. These instructions are referred to as program code, computer usable program code, or computer readable program code that may be read and executed by a processor in processor unit 404. The program code in the different embodiments may be embodied on different physical or tangible computer readable media, such as memory 406 or persistent storage 408.

Program code 416 is located in a functional form on computer readable media 418 that is selectively removable and may be loaded onto or transferred to data processing system 400 for execution by processor unit 404. Program code 416 and computer readable media 418 form computer program product 420 in these examples. In one example, computer readable media 418 may be in a tangible form, such as, for example, an optical or magnetic disc that is inserted or placed into a drive or other device that is part of persistent storage 408 for transfer onto a storage device, such as a hard drive that is part of persistent storage 408. In a tangible form, computer readable media 418 also may take the form of a persistent storage, such as a hard drive, a thumb drive, or a flash memory that is connected to data processing system 400. The tangible form of computer readable media 418 is also referred to as computer recordable storage media. In some instances, computer readable media 418 may not be removable.

Alternatively, program code 416 may be transferred to data processing system 400 from computer readable media 418 through a communications link to communications unit 410 and/or through a connection to input/output unit 412. The communications link and/or the connection may be physical or wireless in the illustrative examples. The computer readable media also may take the form of non-tangible media, such as communications links or wireless transmissions containing the program code.

The different components illustrated for data processing system 400 are not meant to provide architectural limitations to the manner in which different embodiments may be implemented. The different illustrative embodiments may be implemented in a data processing system including components in addition to or in place of those illustrated for data processing system 400. Other components shown in Figure 4 can be varied from the illustrative examples shown.

As one example, a storage device in data processing system 400 is any hardware apparatus that may store data. Memory 406, persistent storage 408, and computer readable media 418 are examples of storage devices in a tangible form.

In another example, a bus system may be used to implement communications fabric 402 and may be comprised of one or more buses, such as a system bus or an input/output bus. Of course, the bus system may be implemented using any suitable type of architecture that provides for a transfer of data between different components or devices attached to the bus system. Additionally, a communications unit may include one or more devices used to transmit and receive data, such as a modem or a network adapter. Further, a memory may be, for example, memory 406 or a cache, such as found in an interface and memory controller hub that may be present in communications fabric 402.

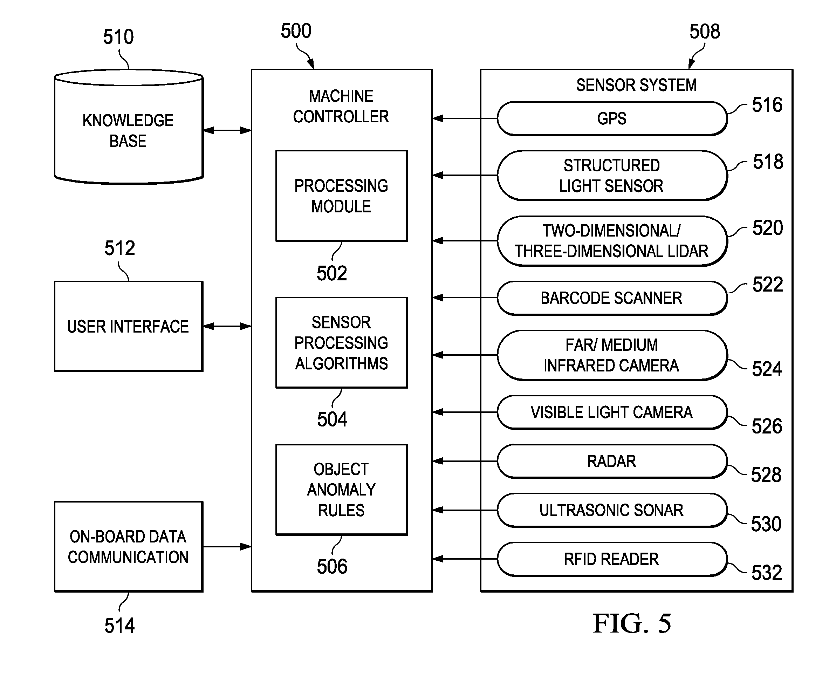

With reference now to Figure 5, a block diagram of functional software components that may be implemented in a machine controller is depicted in accordance with an illustrative embodiment. Machine controller 500 is an example of machine controller 230 in Figure 2. In this example, different functional software components that may be used to control a vehicle are illustrated. The vehicle may be a vehicle, such as vehicle 106 in Figure 1. Machine controller 500 may be implemented in a vehicle, such as vehicle 202 in Figures 2 using a data processing system, such as data processing system 400 in Figure 4. In this example processing module 502, sensor processing algorithms 504, and object anomaly rules 506 are present in machine controller 500. Machine controller 500 interacts with knowledge base 510, user interface 512, on-board data communication 514, and sensor system 508.

Machine controller 500 transmits signals to steering, braking, and propulsion systems to control the movement of a vehicle. Machine controller 500 may also transmit signals to components of a sensor system, such as sensor system 508. For example, in an illustrative embodiment, machine controller 500 transmits a signal to visible light camera 526 of sensor system 508 in order to pan, tilt, or zoom a lens of the camera to acquire different images and perspectives of an operator wearing a garment, such as garment 300 in Figure 3, in an environment around the vehicle. Machine controller 500 may also transmit signals to sensors within sensor system 508 in order to activate, deactivate, or manipulate the sensor itself.

Sensor processing algorithms 504 receives sensor data from sensor system 508 and classifies the sensor data. This classification may include identifying objects that have been detected in the environment. For example, sensor processing algorithms 504 may classify an object as a person, telephone pole, tree, road, light pole, driveway, fence, or some other type of object. The classification may be performed to provide information about objects in the environment. This information may be used to generate a thematic map, which may contain a spatial pattern of attributes. The attributes may include classified objects. The classified objects may include dimensional information, such as, for example, location, height, width, color, and other suitable information. This map may be used to plan actions for the vehicle. The action may be, for example, planning paths to follow an operator wearing a garment, such as garment 300 in Figure 3, in a side following mode or performing object avoidance.

The classification may be done autonomously or with the aid of user input through user interface 512. For example, in an illustrative embodiment, sensor processing algorithms 504 receives data from a laser range finder, such as two dimensional/three dimensional lidar 520 in sensory system 508, identifying points in the environment. User input may be received to associate a data classifier with the points in the environment, such as, for example, a data classifier of "tree" associated with one point, and "fence" with another point. Tree and fence are examples of thematic features in an environment. Sensor processing algorithms 504 then interacts with knowledge base 510 to locate the classified thematic features on a thematic map stored in knowledge base 510, and calculates the vehicle position based on the sensor data in conjunction with the landmark localization. Machine controller 500 receives the environmental data from sensor processing algorithms 504, and interacts with knowledge base 510 in order to determine which commands to send to the vehicle's steering, braking, and propulsion components.

These illustrative examples are not meant to limit the invention in any way. Multiple types of sensors and sensor data may be used to perform multiple types of localization. For example, the sensor data may be used to determine the location of a garment worn by an operator, an object in the environment, or for obstacle detection.

Object anomaly rules 506 provide machine controller 500 instructions on how to operate the vehicle when an anomaly occurs, such as sensor data received by sensor processing algorithms 504 being incongruous with environmental data stored in knowledge base 510. For example, object anomaly rules 506 may include, without limitation, instructions to alert the operator via user interface 514 or instructions to activate a different sensor in sensor system 508 in order to obtain a different perspective of the environment.

Sensor system 508 includes redundant sensors. A redundant sensor in these examples is a sensor that may be used to compensate for the loss and/or inability of another sensor to obtain information needed to control a vehicle. A redundant sensor may be another sensor of the same type (homogenous) and/or a different type of sensor (heterogeneous) that is capable of providing information for the same purpose as the other sensor.

As illustrated, sensor system 508 includes, for example, global positioning system 516, structured light sensor 518, two dimensional/three dimensional lidar 520, barcode scanner 522, far/medium infrared camera 524, visible light camera 526, radar 528, ultrasonic sonar 530, and radio frequency identification reader 532. These different sensors may be used to identify the environment around a vehicle as well as a garment worn by an operator, such as garment 104 in Figure 1 and garment 300 in Figure 3. For example, these sensors may be used to detect the location of worker 102 wearing garment 104 in Figure 1. In another example, these sensors may be used to detect a dynamic condition in the environment. The sensors in sensor system 508 may be selected such that one of the sensors is always capable of sensing information needed to operate the vehicle in different operating environments.

Global positioning system 516 may identify the location of the vehicle with respect to other objects in the environment. Global positioning system 516 may be any type of radio frequency triangulation scheme based on signal strength and/or time of flight. Examples include, without limitation, the Global Positioning System, Glonass, Galileo, and cell phone tower relative signal strength. Position is typically reported as latitude and longitude with an error that depends on factors, such as ionispheric conditions, satellite constellation, and signal attenuation from vegetation.

Structured light sensor 518 emits light in a pattern, such as one or more lines, reads back the reflections of light through a camera, and interprets the reflections to detect and measure objects in the environment. Two dimensional/three dimensional lidar 520 is an optical remote sensing technology that measures properties of scattered light to find range and/or other information of a distant target. Two dimensional/three dimensional lidar 520 emits laser pulses as a beam, than scans the beam to generate two dimensional or three dimensional range matrices. The range matrices are used to determine distance to an object or surface by measuring the time delay between transmission of a pulse and detection of the reflected signal.

Barcode scanner 522 is an electronic device for reading barcodes. Barcode scanner 522 consists of a light source, a lens, and a photo conductor translating optical impulses into electrical ones. Barcode scanner 522 contains decoder circuitry that analyzes image data of a barcode provided by the photo conductor and sends the content of the barcode to the output port of barcode scanner 522.

Far/Medium infrared camera 524 detects heat indicative of a living thing versus an inanimate object. An infrared camera may also form an image using infrared radiation. Far/Medium infrared camera 524 can detect the presence of a human operator when other sensors of sensor system 508 may fail, providing fail-safe redundancy to a vehicle working alongside a human operator.

Visible light camera 526 may be a standard still-image camera, which may be used alone for color information or with a second camera to generate stereoscopic or three-dimensional images. When visible light camera 526 is used along with a second camera to generate stereoscopic images, the two or more cameras may be set with different exposure settings to provide improved performance over a range of lighting conditions. Visible light camera 526 may also be a video camera that captures and records moving images.

Radar 528 uses electromagnetic waves to identify the range, altitude, direction, or speed of both moving and fixed objects. Radar 528 is well known in the art, and may be used in a time of flight mode to calculate distance to an object, as well as Doppler mode to calculate the speed of an object. Ultrasonic sonar 530 uses sound propagation on an ultrasonic frequency to measure the distance to an object by measuring the time from transmission of a pulse to reception and converting the measurement into a range using the known speed of sound. Ultrasonic sonar 530 is well known in the art and can also be used in a time of flight mode or Doppler mode, similar to radar 528. Radio frequency identification reader 532 relies on stored data and remotely retrieves the data using devices called radio frequency identification (RFID) tags or transponders, such as radio frequency identification tag 210 in Figure 2.

Sensor system 508 may retrieve environmental data from one or more of the sensors to obtain different perspectives of the environment. For example, sensor system 508 may obtain visual data from visible light camera 526, data about the distance of the vehicle in relation to objects in the environment from two dimensional/three dimensional lidar 520, and location data of the vehicle in relation to a map from global positioning system 516.

In addition to receiving different perspectives of the environment, sensor system 508 provides redundancy in the event of a sensor failure, which facilitates high-integrity operation of the vehicle. For example, in an illustrative embodiment, if visible light camera 526 is the primary sensor used to identify the location of the operator in side-following mode, and visible light camera 526 fails, radio frequency identification reader 532 will still detect the location of the operator through a radio frequency identification tag on the garment, such as garment 300 in Figure 3, worn by the operator, thereby providing redundancy for safe operation of the vehicle.

Knowledge base 510 contains information about the operating environment, such as, for example, a fixed map showing streets, structures, tree locations, and other static object locations. Knowledge base 510 may also contain information, such as, without limitation, local flora and fauna of the operating environment, current weather for the operating environment, weather history for the operating environment, specific environmental features of the work area that affect the vehicle, and the like. The information in knowledge base 510 may be used to perform classification and plan actions. Knowledge base 510 may be located entirely in machine controller 500 or parts or all of knowledge base 510 may be located in a remote location that is accessed by machine controller 500.

User interface 512 may be, in one illustrative embodiment, presented on a display monitor mounted on a side of a vehicle and viewable by an operator. User interface 512 may display sensor data from the environment surrounding the vehicle, as well as messages, alerts, and queries for the operator. In other illustrative embodiments, user interface 512 may be presented on a remote display on a garment worn by the operator. For example, in an illustrative embodiment, sensor processing algorithms 512 receives data from a laser range finder, such as two dimensional/three dimensional lidar 520, identifying points in the environment. The information processed by sensor processing algorithms 504 is displayed to an operator through user interface 512. User input may be received to associate a data classifier with the points in the environment, such as, for example, a data classifier of "curb" associated with one point, and "street" with another point. Curb and street are examples of thematic features in an environment. Sensor processing algorithms 504 then interacts with knowledge base 510 to locate the classified thematic features on a thematic map stored in knowledge base 510, and calculates the vehicle position based on the sensor data in conjunction with the landmark localization. Machine controller 500 receives the environmental data from sensor processing algorithms 504, and interacts with knowledge base 510 in order to determine which commands to send to the vehicle's steering, braking, and propulsion components.

On-board data communication 514 is an example of communication unit 228 in Figure 2. On-board data communication 514 provides wireless communication between a garment and a vehicle. On-board data communication 514 may also, without limitation, serve as a relay between a first garment and a second garment, a first garment and a remote back office, or a first garment and a second vehicle.

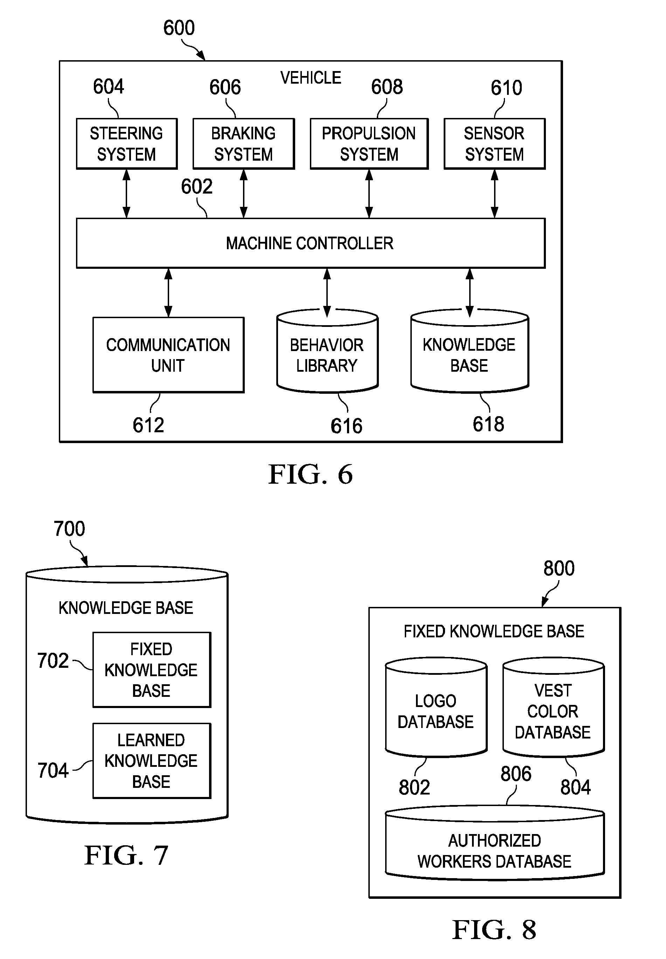

With reference now to Figure 6, a block diagram of components used to control a vehicle is depicted in accordance with an illustrative embodiment. In this example, vehicle 600 is an example of a vehicle, such as vehicle 106 in Figure 1. Vehicle 600 is an example of one implementation of vehicle 202 in Figure 2. In this example, vehicle 600 includes machine controller 602, steering system 604, braking system 606, propulsion system 608, sensor system 610, communication unit 612, behavior library 616, and knowledge base 618.

Machine controller 602 may be, for example, a data processing system, such as data processing system 400 in Figure 4, or some other device that may execute processes to control movement of a vehicle. Machine controller 602 may be, for example, a computer, an application integrated specific circuit, and/or some other suitable device. Different types of devices and systems may be used to provide redundancy and fault tolerance. Machine controller 602 may execute processes to control steering system 604, braking system 606, and propulsion system 608 to control movement of the vehicle. Machine controller 602 may send various commands to these components to operate the vehicle in different modes of operation. These commands may take various forms depending on the implementation. For example, the commands may be analog electrical signals in which a voltage and/or current change is used to control these systems. In other implementations, the commands may take the form of data sent to the systems to initiate the desired actions.

Steering system 604 may control the direction or steering of the vehicle in response to commands received from machine controller 602. Steering system 604 may be, for example, an electrically controlled hydraulic steering system, an electrically driven rack and pinion steering system, an Ackerman steering system, a skid-steer steering system, a differential steering system, or some other suitable steering system.

Braking system 606 may slow down and/or stop the vehicle in response to commands from machine controller 602. Braking system 606 may be an electrically controlled steering system. This steering system may be, for example, a hydraulic braking system, a friction braking system, or some other suitable braking system that may be electrically controlled.

In these examples, propulsion system 608 may propel or move the vehicle in response to commands from machine controller 602. Propulsion system 608 may maintain or increase the speed at which a vehicle moves in response to instructions received from machine controller 602. Propulsion system 608 may be an electrically controlled propulsion system. Propulsion system 608 may be, for example, an internal combustion engine, an internal combustion engine/electric hybrid system, an electric engine, or some other suitable propulsion system.

Sensor system 610 may be a set of sensors used to collect information about the environment around a vehicle. In these examples, the information is sent to machine controller 602 to provide data in identifying how the vehicle should move in different modes of operation. In these examples, a set refers to one or more items. A set of sensors is one or more sensors in these examples.

Communication unit 612 may provide multiple redundant communications links and channels to machine controller 602 to receive information. The communication links and channels may be heterogeneous and/or homogeneous redundant components that provide fail-safe communication. This information includes, for example, data, commands, and/or instructions. Communication unit 612 may take various forms. For example, communication unit 612 may include a wireless communications system, such as a cellular phone system, a Wi-Fi wireless system, a Bluetooth wireless system, and/or some other suitable wireless communications system. Further, communication unit 612 also may include a communications port, such as, for example, a universal serial bus port, a serial interface, a parallel port interface, a network interface, and/or some other suitable port to provide a physical communications link. Communication unit 612 may be used to communicate with a remote location or an operator.

Behavior library 616 contains various behavioral processes specific to machine coordination that can be called and executed by machine controller 602. Behavior library 616 may be implemented in a remote location, such as garment 104 in Figure 1, or in one or more vehicles. In one illustrative embodiment, there may be multiple copies of behavior library 616 on vehicle 600 in order to provide redundancy.

Knowledge base 618 contains information about the operating environment, such as, for example, a fixed map showing streets, structures, tree locations, and other static object locations. Knowledge base 618 may also contain information, such as, without limitation, local flora and fauna of the operating environment, current weather for the operating environment, weather history for the operating environment, specific environmental features of the work area that affect the vehicle, and the like. The information in knowledge base 618 may be used to perform classification and plan actions. Knowledge base 618 may be located entirely in vehicle 600 or parts or all of knowledge base 618 may be located in a remote location that is accessed by machine controller 602.

With reference now to Figure 7, a block diagram of a knowledge base is depicted in accordance with an illustrative embodiment. Knowledge base 700 is an example of a knowledge base component of a machine controller, such as knowledge base 618 of vehicle 600 in Figure 6. For example, knowledge base 700 may be, without limitation, a component of a navigation system, an autonomous machine controller, a semi-autonomous machine controller, or may be used to make management decisions regarding work-site activities and coordination activities. Knowledge base 700 includes fixed knowledge base 702 and learned knowledge base 704.

Fixed knowledge base 702 contains static information about the operating environment of a vehicle. Types of information about the operating environment of a vehicle may include, without limitation, a fixed map showing streets, structures, trees, and other static objects in the environment; stored geographic information about the operating environment; and weather patterns for specific times of the year associated with the operating environment.

Fixed knowledge base 702 may also contain fixed information about objects that may be identified in an operating environment, which may be used to classify identified objects in the environment. This fixed information may include attributes of classified objects, for example, an identified object with attributes of tall, narrow, vertical, and cylindrical, may be associated with the classification of "telephone pole." Fixed knowledge base 702 may further contain fixed work-site information. Fixed knowledge base 702 may be updated based on information from learned knowledge base 706.

Fixed knowledge base 702 may also be accessed with a communications unit, such as communications unit 612 in Figure 6, to wirelessly access the Internet. Fixed knowledge base 702 may dynamically provide information to a machine control process which enables adjustment to sensor data processing, site-specific sensor accuracy calculations, and/or exclusion of sensor information. For example, fixed knowledge base 702 may include current weather conditions of the operating environment from an online source. In some examples, fixed knowledge base 702 may be a remotely accessed knowledge base. This weather information may be used by machine controller 602 in Figure 6 to determine which sensors to activate in order to acquire accurate environmental data for the operating environment. Weather, such as rain, snow, fog, and frost may limit the range of certain sensors, and require an adjustment in attributes of other sensors in order to acquire accurate environmental data from the operating environment. Other types of information that may be obtained include, without limitation, vegetation information, such as foliage deployment, leaf drop status, and lawn moisture stress, and construction activity, which may result in landmarks in certain regions being ignored.

Learned knowledge base 704 may be a separate component of knowledge base 700, or alternatively may be integrated with fixed knowledge base 702 in an illustrative embodiment. Learned knowledge base 704 contains knowledge learned as the vehicle spends more time in a specific work area, and may change temporarily or long-term depending upon interactions with fixed knowledge base 702 and user input. For example, learned knowledge base 704 may detect the absence of a tree that was present the last time it received environmental data from the work area. Learned knowledge base 704 may temporarily change the environmental data associated with the work area to reflect the new absence of a tree, which may later be permanently changed upon user input confirming the tree was in fact cut down. Learned knowledge base 704 may learn through supervised or unsupervised learning.

With reference now to Figure 8, a block diagram of a fixed knowledge base is depicted in accordance with an illustrative embodiment. Fixed knowledge base 800 is an example of fixed knowledge base 702 in Figure 7.

Fixed knowledge base 800 includes logo database 802, vest color database 804, and authorized workers database 806. Logo database 802, vest color database 804, and authorized workers database 806 are examples of stored information used by a machine controller to authenticate a worker wearing a garment before initiating a process or executing a task.

Logo database 802 stores information about recognizable logos associated with vehicle operation. In an illustrative example, a machine controller, such as machine controller 500 in Figure 5, may search for a logo on a garment worn by an operator using a visible light camera on the sensor system of the vehicle. Once the machine controller detects a logo, the machine controller interacts with fixed knowledge base 800 to compare the logo detected with the approved or recognizable logos stored in logo database 802. If the logo matches an approved or recognizable logo, the vehicle may initiate a process or execute a task, such as following the operator wearing the garment with the approved or recognizable logo. In another illustrative embodiment, if the logo detected is not found in logo database 802, the vehicle may fail to initiate a process or execute a task.

Vest color database 804 stores information about the approved or recognizable vest colors that are associated with the vehicle. Authorized worker database 806 may include information about authorized workers including, without limitation, physical description and employee identification.

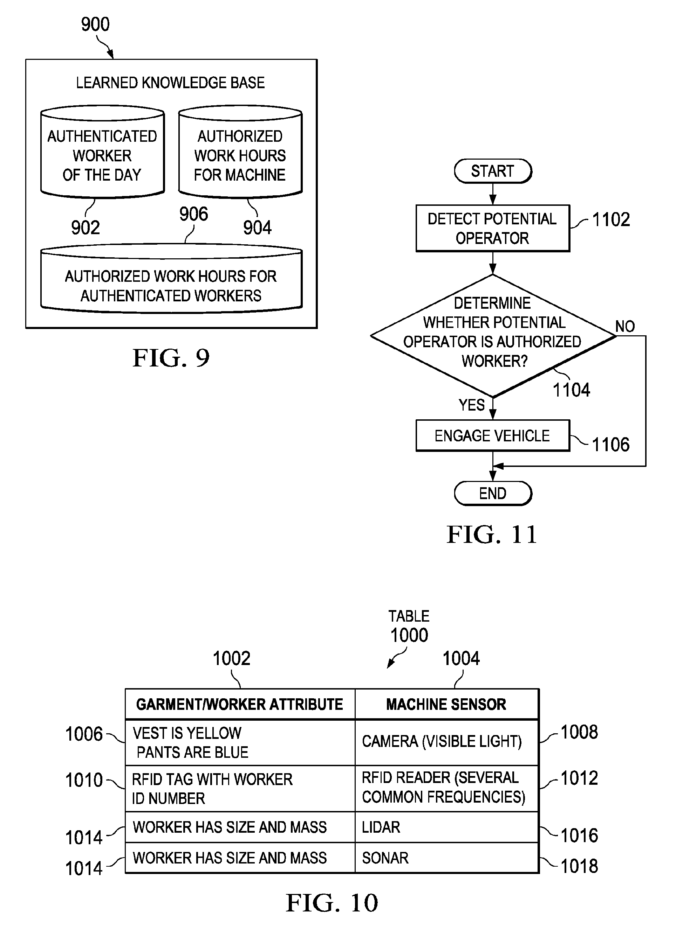

The illustration of fixed knowledge base 800 is not meant to imply physical or architectural limitations to the manner in which different illustrative embodiments may be implemented. For example, in other embodiments, other components may be used in addition to or in place of the ones illustrated for fixed knowledge base 800. For example, in other embodiments, fixed knowledge base 800 may not have logo database 802. In still other illustrative embodiments, fixed knowledge base 800 may include additional databases of identifying information, such as a serial number database for robotic operators. With reference now to Figure 9, a block diagram of a learned knowledge base is depicted in accordance with an illustrative embodiment. Learned knowledge base 900 is an example of learned knowledge base 704 in Figure 7.

Learned knowledge base 900 includes authenticated worker of the day 902, authorized work hours for machine 904, and authorized work hours for authenticated workers 906. Authenticated worker of the day 902, authorized work hours for machine 904, and authorized work hours for authenticated workers 906 are examples of stored information used by a machine controller to authenticate an operator before initiating a process or executing a task.

Authenticated worker of the day 902 may include identification information for individual operators and information about which day or days of the week a particular operator is authorized to work. Authorized work hours for machine 904 may include parameters indicating a set period of time, a set time of day, or a set time period on a particular day of the week or calendar date on which the vehicle is authorized to work. Authorized work hours for authenticated workers 906 may include specific hours in a day, a specific time period within a day or calendar date, or specific hours in a calendar date during which an operator is authorized to work with a vehicle. In an illustrative embodiment, if an operator wearing a garment, such as garment 104 in Figure 1, attempts to initiate an action or execute a process using a vehicle, such as vehicle 106 in Figure 1, the machine controller, such as machine controller 602 in Figure 6, will interact with learned knowledge base 900 to determine whether the operator is an authenticated worker of the day, and if the current request is begin made during authorized work hours for both the vehicle and the authenticated worker.

The illustration of learned knowledge base 900 is not meant to imply physical or architectural limitations to the manner in which different illustrative embodiments may be implemented. For example, in other embodiments, other components may be used in addition to or in place of the ones illustrated for learned knowledge base 900. For example, in other embodiments, learned knowledge base 900 may not have authorized work hours for machine 904. In still other illustrative embodiments, learned knowledge base 900 may include additional databases of authorization information.

With reference now to Figure 10, a block diagram of a format in a knowledge base used to select sensors for use in detecting and localizing a garment and/or worker is depicted in accordance with an illustrative embodiment. This format may be used by machine controller 500 in Figure 5, using a sensor system, such as sensor system 508 in Figure 5.

The format is depicted in table 1000 illustrating heterogeneous sensor redundancy for localization of the garment and/or worker. Garment/worker attribute 1002 may be any type of distinguishing or recognizable attribute that can be detected by a sensor system. Examples of distinguishing or recognizable attributes include, without limitation, garment color, garment pattern, garment size, radio frequency identification tag, visible logo, barcode, worker identification number, worker size, worker mass, physical attributes of worker, and the like. Machine sensor 1004 may be any type of sensor in a sensor system, such as sensor system 508 in Figure 5.

In an illustrative embodiment, where garment/worker attribute 1002 is a yellow vest and blue pants 1006, visible light camera 1008 may detect the color of the vest and pants to localize the position of the worker wearing yellow vest and blue pants 1006. However, in an operating environment with low visibility, visible light camera 1008 may be unable to detect yellow vest and blue pants 1006. In a situation with low visibility, for example, radio frequency identification tag with worker identification number 1010 may be detected by radio frequency identification reader 1012 located on the vehicle. Worker size and mass 1014 may be detected by lidar 1016 or by sonar 1018. High integrity detection and localization is provided by the redundancy of heterogeneous sensors and garment/worker attributes.

The illustration in table 1000 is not meant to imply physical or architectural limitations to the manner in which different illustrative embodiments may be implemented. For example, in other embodiments, there may be two or more radio frequency identification tags with worker identification number 1010 that are detectable by radio frequency identification reader 1012. In this illustrative embodiment, where two or more radio frequency identification tags are detectable, where one radio frequency identification tag fails, radio frequency identification reader 1012 may still be able to detect the one or more other radio frequency identification tags located on the garment. This is an example of homogeneous redundancy used alongside heterogeneous redundancy provided for detecting and localizing the wearer of a garment, such as garment 300 in Figure 3.

With reference now to Figure 11, a flowchart illustrating a process for engaging a vehicle is depicted in accordance with an illustrative embodiment. This process may be executed by processing module 502 in Figure 5.

The process begins by detecting a potential operator (step 1102). The process determines whether the potential operator is an authorized worker (step 1104). An authorized worker is an operator who is allowed to use the vehicle or who is allowed to be in proximity of the vehicle while it is operating. An authorized worker may also be an operator who is allowed to use the vehicle at the time the operator is requesting to use the vehicle. This determination may be made using a knowledge base, such as knowledge base 510 in Figure 5, to retrieve information on authorized workers and authorized workers for the current time and day. If the process determines the potential operator is not an authorized worker, the process terminates. If the process determines the potential operator is an authorized worker, the process then engages the vehicle (step 1106), with the process terminating thereafter. Engaging the vehicle may be, without limitation, starting the vehicle engine, propelling the vehicle, initiating a vehicle task or action, and the like. Determining worker authorization before allowing a vehicle to engage in a task or action provides safeguards against a vehicle being used by unauthorized personnel or for unauthorized work or actions.

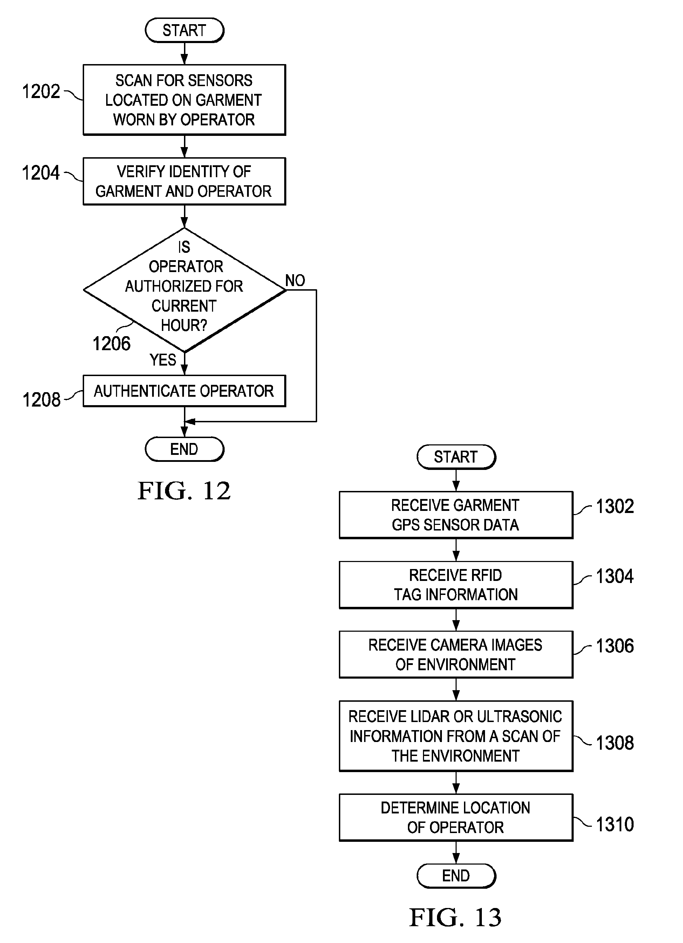

With reference now to Figure 12, a flowchart illustrating a process for authenticating an operator is depicted in accordance with an illustrative embodiment. This process may be executed by processing module 502 in Figure 5. Some or all data used for operator identification and authentication may be encrypted.

The process begins by scanning for sensors located on a garment worn by an operator (step 1202). Sensors may include, without limitation, radio frequency identification tags, global positioning system sensors, a barcode, as well as attributes of the garment, such as, without limitation, color, size, pattern, logo, and the like. Next, the process verifies the identity of the garment and the operator (step 1204). The verification may be executed using different aspects of a knowledge base, such as logo database 802, vest color database 804, and authorized worker database 806 of Figure 8. The authorized worker database 806 may require entry of a password or a number from a number generating means for further authentication. The process then determines whether the operator is authorized for the current hour (step 1206), using aspects of a knowledge base, such as authenticated worker of the day 902, authorized worker hours for machine 904, and authorized work hours for authenticated workers 906 of Figure 9. If the operator is not authorized for the current hour, the process terminates. If the operator is authorized for the current hour, the process then authenticates the operator (step 1208), with the process terminating thereafter.

The process illustrated in Figure 12 is not meant to imply physical or architectural limitations. For example, the process may scan for one or more garments worn by one or more potential operators in a work environment. The process may authenticate more than one operator as authorized to work with the machine or during the current hour. In an illustrative embodiment, multiple authenticated operators may have varying degrees of control over a vehicle or machine. For example, each authenticated operator may have an emergency stop control feature, but one authenticated operator may have the authority to steer, change gears, throttle, and brake within a work area, while another authenticated operator may have authority to move the vehicle off the work site in addition to the preceding rights. The examples presented are different illustrative embodiments in which the present invention may be implemented.

With reference now to Figure 13, a flowchart illustrating a process for localization of an operator by a vehicle is depicted in accordance with an illustrative embodiment. This process may be executed by processing module 502 in Figure 5.

The process begins by receiving garment global positioning system data (step 1302). The data may be received from a global positioning system sensor on the garment of a worker. Next, the process receives radio frequency identification tag information (step 1304), from one or more radio frequency identification tags located on a garment worn by an operator. The process then receives camera images of the environment (step 1306), which may include images of the operator as well as the operating environment around the vehicle. The process receives lidar or ultrasonic information from a scan of the environment (step 1308), using a sensor system, such as sensor system 508 in Figure 5. The process then determines the location of the operator (step 1310) based on the global positioning data, the radio frequency identification tag information, the images of the environment, and the lidar and/or ultrasonic information received, with the process terminating thereafter.

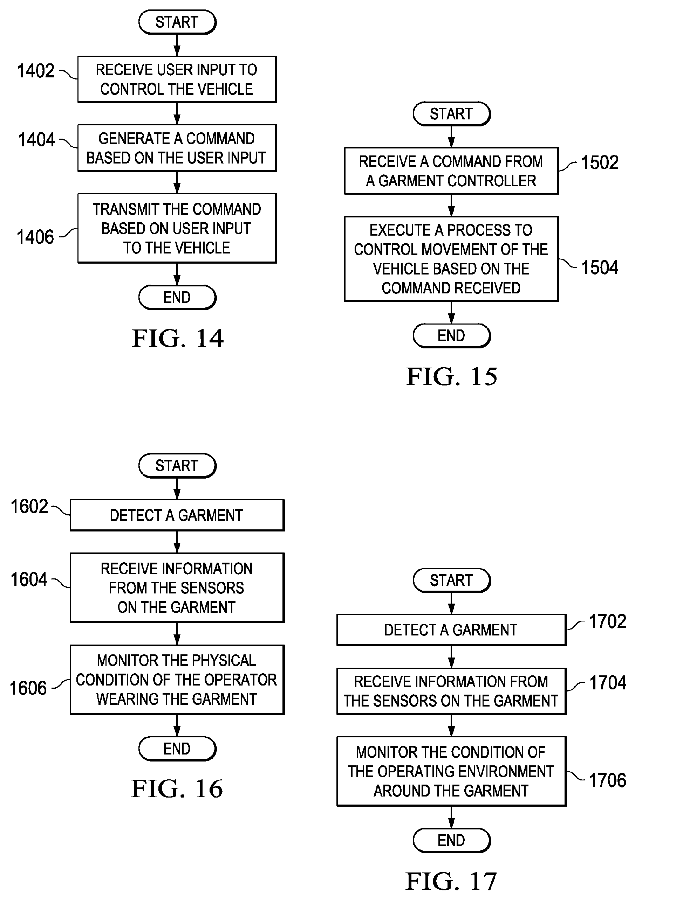

With reference now to Figure 14, a flowchart illustrating a process for controlling a vehicle with a garment is depicted in accordance with an illustrative embodiment. This process may be executed by controller 216 of garment 200 in Figure 2.

The process begins by receiving user input to control the vehicle (step 1402). User input may be received using a user interface, such as interface 218 in Figure 2. Next, the process generates a command based on the user input (step 1404). Commands are generated by a controller, such as controller 216 in Figure 2. The controller interacts with the user interface to obtain the user input received at the user interface and translate the user input into a machine command. The process then transmits the command based on the user input to the vehicle (step 1406), with the process terminating thereafter.

With reference now to Figure 15, a flowchart illustrating a process for receiving commands from a garment to control a vehicle is depicted in accordance with an illustrative embodiment. This process may be executed by machine controller 230 on vehicle 202 in Figure 2.

The process begins by receiving a command from a garment controller (step 1502), such as controller 216 on garment 200 in Figure 2. The command received is in the form of a vehicle control command generated from user input received at the garment, such as garment 200 in Figure 2. The command received may by, for example, without limitation, a command to turn the vehicle, propel the vehicle, bring the vehicle to a halt, apply the brakes of the vehicle, follow a leader wearing a garment, follow a route, execute a behavior, and the like. The process then executes a process to control movement of the vehicle based on the command received (step 1504), with the process terminating thereafter. In an illustrative embodiment, the process is executed by a machine controller, such as machine controller 230 in Figure 2, using high integrity control software to control the mechanical systems of the vehicle, such as the steering, braking, and propulsion systems. In an illustrative embodiment, if the command received is a command to turn the vehicle, the machine controller may send a signal to the steering component of the mechanical system of the vehicle to turn the vehicle in the direction according to the command received.

With reference now to Figure 16, a flowchart illustrating a process for monitoring the condition of an operator is depicted in accordance with an illustrative embodiment. This process may be executed by processing module 502 in Figure 5.

The process begins by detecting a garment (step 1602). The garment may be detected using a sensor system, such as sensor system 508 in Figure 5, to detect a number of different sensors and/or attributes located on the garment. For example, in an illustrative embodiment, the process may detect radio frequency identification tags on the garment, as well as the color of the garment and a visible logo on the garment.

Next, the process receives information from the sensors on the garment (step 1604). In an illustrative embodiment, the information received may be from sensors that monitor the well-being of the wearer of the garment, such as redundant sensors 322 in Figure 3. Examples of redundant sensors 322 may include, without limitation, a heart-rate monitor, a blood pressure sensor, a CO₂ monitor, a body temperature sensor, and the like.

In another illustrative embodiment, the information received may be from radio frequency identification tags, such as radio frequency identification tag 308 in Figure 3, that provide localization information and information about the orientation of the wearer of the garment. For example, orientation of the wearer of the garment may be information about the orientation of the operator in relation to the autonomous vehicle and/or the orientation of the operator in relation to the operating environment surface. In another illustrative embodiment, information about the orientation of the operator in relation to the operating environment surface may indicate whether the operator is down or prostrate, for example, due to physical distress in a human or animal operator, or systems failure in a robotic or autonomous vehicle operator. The process then monitors the physical condition of the operator wearing the garment (step 1602), with the process terminating thereafter.

The illustration in process 1600 is not meant to imply physical or architectural limitations to the manner in which different illustrative embodiments may be implemented. For example, in other embodiments, other steps and/or components may be used in addition to or in place of the ones illustrated in process 1600. For example, in other embodiments, information received from sensors may include further physical information about the operator wearing the garment, such as systems integrity of a robotic operator. Monitoring the physical condition of the operator is another aspect of fail-safe operations. With reference now to Figure 17, a flowchart illustrating a process for monitoring the condition of the operating environment is depicted in accordance with an illustrative embodiment. This process may be executed by processing module 502 in Figure 5.

The process begins by detecting a garment (step 1702). The garment may be detected using a sensor system, such as sensor system 508 in Figure 5, to detect a number of different sensors and/or attributes located on the garment. For example, in an illustrative embodiment, the process may detect radio frequency identification tags on the garment, as well as the pattern of the garment and the mass of the worker wearing the garment.

Next, the process receives information from the sensors on the garment (step 1704). In an illustrative embodiment, the information received may be from sensors that monitor the environment around the garment, such as redundant sensors 322 in Figure 3. Examples of redundant sensors 322 may include, without limitation, an environmental temperature sensor, a hazardous chemical sensor, a toxic gas sensor, and the like. The process then monitors the condition of the operating environment around the garment (step 1706), with the process terminating thereafter.

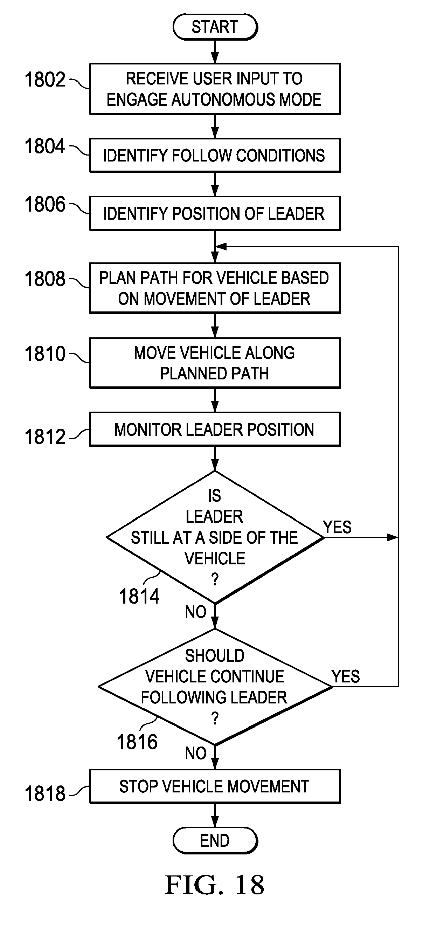

With reference now to Figure 18, a flowchart illustrating a process for side-following is depicted in accordance with an illustrative embodiment. This process may be executed by machine controller 500 in Figure 5. The process begins by receiving user input to engage autonomous mode (step 1802). The user input may be received from a user interface on a garment, such as interface 218 on garment 200 in Figure 2. The process identifies following conditions (step 1804) and identifies the position of the leader (step 1806). Follow conditions are stored as part of a side-following machine behavior in behavior library 616 in Figure 6. Follow conditions may be conditions, such as, without limitation, identifying an authorized worker in the area around the vehicle, detecting the authorized worker towards the front of the vehicle, detecting the authorized worker at a side of the vehicle, detecting that the position of the authorized worker is changing towards the next location in a planned path, and the like. The leader may be an authorized worker identified through various means including, without limitation, a radio frequency identification tag located on the garment worn the authorized worker or user input by an authorized worker identifying the worker as a leader. Next, the process plans a path for the vehicle based on movement of the leader (step 1808) and moves the vehicle along the planned path (step 1810). Machine controller 500 in Figure 5 plans the path for the vehicle based on movement of the worker detected by a sensor system, such as sensor system 508 in Figure 5. Sensor system 508 sends sensor information to sensor processing algorithms 504 in machine controller 500. Machine controller 500 uses the sensor information to move the vehicle along the planned path following the worker. Next, the process continues to monitor the leader position (step 1812). While monitoring the position of the leader, the process determines whether the leader is still at a side of the vehicle (step 1814). The process may determine the position of the leader by using sensors of sensor system 508 in Figure 5.

If the leader is still at a side of the vehicle, the process continues on the planned path for the vehicle based on movement of the leader (step 1808). If the leader is no longer at a side of the vehicle, the process then determines whether the vehicle should continue following the leader (step 1816). If the process determines that the vehicle should continue following the leader, it returns to the planned path for the vehicle based on movement of the leader (step 1808). However, if the process determines that the vehicle should not continue following the leader, the process stops vehicle movement (step 1818), with the process terminating thereafter.

## Claims

1. An apparatus for providing an interface between a machine, for example a vehicle, and an operator of the machine, the apparatus comprising:
a garment capable of being worn by the operator;
at least one localization device, connected to the garment, capable of being detected by the machine for determining a location of the garment; and
a controller capable of sending a control signal from the garment to the machine to control an operation of the machine.

2. The apparatus of claim 1, further comprising:
a separate localization device on at least two, preferably each of a front, back, right, and left side of the garment for determining an orientation of the operator.

3. The apparatus of claim 1, wherein the controller is a touch sensitive area on the garment.

4. The apparatus of claim 1, further comprising:
at least one sensor connected to the garment for detecting at least one physical condition of the operator.

5. The apparatus of claim 1, further comprising:
a wireless communication sender and receiver means connected to the garment for transmitting information to the machine and receiving information from the machine.

6. The apparatus of claim 1, further comprising:
a plurality of different types of attributes associated with the garment and detectable by a plurality of different types of sensors located on the machine, the plurality of different types of attributes preferably including at least one of garment material color, garment size, garment pattern, a visual logo, a barcode, and radio frequency identification tags, the attributes in particular detectable by at least one of a global positioning system, a light sensor, a two dimensional/three dimensional model lidar, a barcode scanner, a far/medium infrared camera, a visible light camera, a radar, an ultrasonic sonar, and a radio frequency identification reader.

7. A garment comprising a material of a color that is distinguishable, by a computer, from a work environment; and at least one radio frequency identification tag.

8. The garment of claim 7, wherein the at least one radio frequency identification tag is used for identification, authentication, and localization determination by a nearby vehicle, the nearby vehicle preferably comprising a radio frequency identification reader for sensing the at least one radio frequency identification tag on the garment.

9. The garment of claim 7, further comprising:
at least one radio frequency identification tag in a plurality of radio frequency identification tags located on the front of the garment;
at least one radio frequency identification tag in the plurality of radio frequency identification tags located on the left side of the garment;
at least one radio frequency identification tag in the plurality of radio frequency identification tags located on the right side of the garment; and
at least one radio frequency identification tag in the plurality of radio frequency identification tags located on the back of the garment, wherein the plurality of radio frequency identification tags located at different locations on the garment are used for determining the orientation of an operator wearing the garment.

10. The garment of claim 7, further comprising wireless communication means for transmitting information between the garment and the nearby vehicle, the wireless communications means preferably further comprising:
a plurality of different types of communication channels;
an integrated microphone; and
an integrated speaker.

11. The garment of claim 10, further comprising:
a plurality of different types of sensors for monitoring an operating environment, in particular at least one of an environmental temperature sensor, a chemical sensor, a hazardous substance sensor, a radiation sensor, a vibration sensor, and an oxygen sensor and/or
a plurality of different types of sensors for monitoring a physical condition of an operator wearing the garment, in particular including at least one of a heart rate sensor, a blood sugar sensor, a dehydration sensor, and a body temperature sensor and/or
a barcode readable by a barcode scanner and/or
a battery powering electronic components of the garment and means for recharging the battery and/or
a display for showing operating information of the nearby vehicle and/or
a touch sensitive area for remote control of the nearby vehicle, wherein the touch sensitive area uses the wireless communications means to transmit commands to the nearby vehicle, the touch sensitive area preferably comprising an emergency stop control area for remote control of the nearby vehicle and/ propulsion, steering, and braking control areas for remote control of the nearby vehicle.

12. A method, implemented by a machine, for interacting with a nearby operator, the method comprising detecting at least two attributes of a garment worn by the operator using a plurality of different types of sensors and receiving a control operation for the machine from the garment.

13. The method of claim 12, wherein the at least two attributes include at least one of an image, a color, a pattern, a size of the garment, a logo, a radio frequency identification tag, and a barcode and/or wherein the plurality of different types of sensors include at least two of a global positioning system, a light sensor, a two dimensional/three dimensional model lidar, a barcode scanner, a far/medium infrared camera, a visible light camera, a radar, an ultrasonic sonar, and a radio frequency identification reader and/or the method comprises identifying a condition of the operating environment using the information, for example receiving at least one of environmental temperature information, receiving toxic gas level information and receiving harmful chemical level information.

14. The method of claim 12, further comprising:
receiving information from the garment;
processing the information received from the garment; and
transmitting a message based on the information processed to a user display on the garment,
for example monitoring a physical condition of the operator using the information; and monitoring an orientation of the operator using the information, wherein monitoring the physical condition of the operator using the information can comprise:
receiving body temperature information;
receiving heart rate information; and
receiving blood pressure information,
wherein monitoring the orientation of the operator can comprise:
receiving the information about the orientation of the operator in relation to the autonomous vehicle; and
receiving the information about the orientation of the operator in relation to the operating environment surface,
wherein the orientation of the operator in relation to the operating environment surface indicates whether the operator is down.

15. A method for remotely controlling a machine, the method comprising emitting a radio frequency from a garment worn by an operator; and transmitting operating commands to a machine controller of the machine from the garment, the operating commands in particular include at least one of propulsion commands, steering commands, braking commands, and emergency stop commands.

16. A method for monitoring for a number of operators of an autonomous vehicle, the method comprising:
identifying a location of a first operator wearing a first garment having a number of localization devices capable of being detected by the autonomous vehicle and having a first controller;
identifying a location of a second operator wearing a second garment having the number of localization devices capable of being detected by the autonomous vehicle and having a second controller; and
performing operations based on the location of the first operator and the second operator and a control signal generated by the first controller, and preferably:
detecting a person with an autonomous vehicle; and
identifying the person as a third operator if the person is wearing the garment, wherein the step of identifying the person as the third operator if the person is wearing the garment preferably comprises:
determining whether the person is authorized to be the third operator; and
identifying the person as the third operator in response to a determination that the person is authorized to be the third operator, and wherein determining whether the person is authorized to be the third operator preferably further comprises:
receiving an authentication value, wherein the authentication value is at least one of an authentication code, a radio frequency identification tag, a barcode, facial recognition, physical description recognition, and logo identification.
